# DEMANDE DE BREVET EUROPEEN

(11) **EP 0 571 306 A1**
(43) Date de publication de la demande: **24.11.1993**
(21) Numéro de dépôt: 93430007.0
(22) Date de dépôt: 21.05.1993
(51) Int. Cl.: A61B 17/22

(54) **Dispositif et procédé pour enlever des dépôts sur les parois de passages**

(30) Priorité: 22.05.1992 US 886878
(71) Demandeur: LASER MEDICAL TECHNOLOGY, Inc., San Clemente, California 92672 (US)
(72) Inventeur: Levy, Guy, Tustin, California 92680 (US)
(74) Mandataire: Marek, Pierre

(57) **Abrégé**

Dispositif pour désintégrer un dépôt sur une surface intérieure d'un passage tubulaire susceptible d'être endommagé lorsqu'il est exposé à la chaleur, caractérisé en ce qu'il comprend :
- un tube creux (8, 8') qui présente une ouverture et qui peut être inséré dans le passage (2), de sorte que l'ouverture soit placée à proximité du dépôt ;
- un corps souple et gonflable (10, 10'), constitué d'une feuille de matière fixée audit tube, de manière à fermée ladite ouverture ;
- une fibre optique (12) placée dans ledit tube (8,8') et présentant une extrémité distale à proximité dudit corps gonflable (10, 10') ;
- des moyens (22) pour introduire un volume de liquide dans ledit tube (8, 8') et l'amener en contact avec ledit corps gonflable (10, 10') , à une pression suffisante pour gonfler ce dernier de manière à le mettre en contact avec ledit dépôt (4), et
- des moyens (20) pour envoyer une succession d'impulsions d'un rayon laser à ladite fibre optique (12).

## Description

La présente invention concerne l'élimination de dépôts qui se forment sur les parois intérieures de passages, et en particulier l'enlèvement, par désintégration, de dépôts en plaques, ou athéromes, qui se forment sur les parois internes des vaisseaux sanguins.

Le document US-A-5.116.227 dévoile un procédé et un dispositif pour nettoyer et élargir des passages, y compris pour enlever des dépôts en plaques dans des vaisseaux sanguins. Selon le contenu de ce document qui est incorporé à la présente par référence, un rayonnement laser sous forme d'impulsions est envoyé à une fibre optique qui présente une extrémité distale immergée dans un liquide à l'emplacement d'un tel dépôt. Une succession d'impulsions d'un rayonnement laser ayant un niveau énergétique par pulsation choisi, est envoyée à la fibre optique. L'extrémité distale de la fibre peut être conformée de manière à focaliser le rayonnement laser en un point situé dans le liquide, au-delà de l'extrémité distale de la fibre. L'énergie du rayon laser pulsé concentrée dans le liquide provoque une cavitation de vapeur au sein du liquide, qui se traduit par l'implosion de bulles de gaz, cette implosion entraînant une érosion du dépôt exposé au phénomène de cavitation.

Suivant un tel procédé, le nettoyage de la paroi du passage est exécuté mécaniquement, par l'agitation vigoureuse subie par le liquide. Toutefois, l'énergie du rayon laser est nécessairement absorbée par le liquide, et peut donc provoquer un chauffage important du liquide et des parois des vaisseaux.

Dans des situations où le liquide soumis à cavitation peut être réapprovisionné en permanence, afin d'évacuer la chaleur générée par le rayonnement laser, il est possible d'éviter un endommagement thermique inacceptable des parois des passages. Toutefois, dans d'autres cas, tel que celui de l'élimination des dépôts formés sur les parois des vaisseaux sanguins, le liquide soumis à cavitation est le sang lui-même, qui ne peut être refroidi que par des procédés complexes, et qui peut être endommagé par l'énergie émise par le rayonnement laser.

La présente invention a pour but d'enlever des dépôts formés sur les parois d'un passage, en utilisant l'énergie fournie par les impulsions d'un rayon laser, avec une sécurité accrue.

L'invention a pour autre but de dissiper efficacement la chaleur générée par des opérations du type décrit précédemment, dans un vaisseau sanguin.

Un autre objet de l'invention est de préserver le sang présent dans un vaisseau sanguin des dommages thermiques dus à la chaleur produite par les impulsions d'un rayonnement laser.

Selon l'invention, les buts ci-dessus, et d'autres encore, sont atteints grâce à la prévision d'un dispositif permettant de désintégrer un dépôt sur une surface intérieure d'un passage tubulaire susceptible d'être endommagé lorsqu'il est exposé à la chaleur, et comprenant : un tube creux présentant une ouverture et qui peut être inséré dans le passage de sorte que cette ouverture soit placée adjacente au dépôt ; un corps souple et gonflable, constitué d'une mince feuille de matière fixée au tube de manière à fermer l'ouverture ; une fibre optique placée dans le tube et présentant une extrémité distale à proximité du corps gonflable ; des moyens pour introduire une masse liquide dans le tube et l'amener au contact du corps gonflable, à une pression suffisante pour gonfler ledit corps de manière à l'amener en contact avec le dépôt ; et des moyens pour envoyer une succession d'impulsions d'un rayon laser à la fibre optique.

Les objectifs de l'invention sont atteints, en outre, par un procédé de mise en oeuvre du dispositif décrit précédemment, consistant à insérer le tube creux susmentionné, dans un passage tubulaire, de telle sorte que le corps souple et gonflable soit aligné avec le dépôt à éliminer, à introduire un liquide de refroidissement dans le tube, de telle sorte que le liquide remplisse l'espace enveloppé par le corps gonflable et gonfle ce corps de manière à le presser contre la surface du dépôt, et à envoyer une succession d'impulsions d'un rayon laser à l'extrémité distale de la fibre optique, de manière à transformer une partie du liquide remplissant le corps gonflable en vapeur et à provoquer une cavitation de la vapeur au sein de ce liquide.

Les buts, caractéristiques et avantages ci-dessus et d'autres encore, ressortiront mieux de la description qui suit et des dessins annexés dans lequels :

La figure 1 est une vue en coupe transversale d'un premier mode de réalisation d'un dispositif conforme à l'invention.

La figure 2 est une vue similaire à celle de la figure 1 et illustrant un deuxième mode de réalisation du dispositif selon l'invention.

On se reporte auxdits dessins pour décrire des exemples avantageux de mise en oeuvre du procédé et de réalisation du dispositif de l'invention.

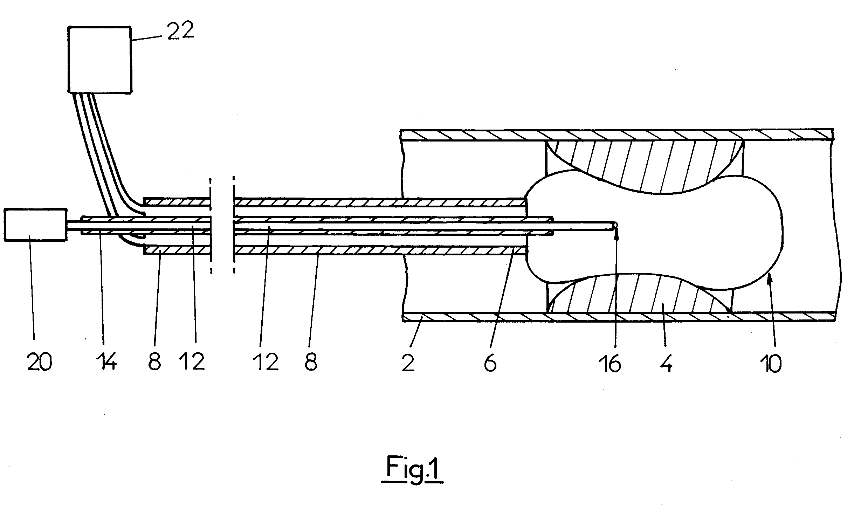

La figure 1 illustre un dispositif conforme à l'invention, inséré dans un vaisseau sanguin 2 sur la paroi interne duquel un dépôt 4 s'est développé. Ce dépôt peut être une plaque, ou un athérome. A défaut d'intervention médicale, ce dépôt poursuivrait sa croissance jusqu'à obstruer complètement le vaisseau sanguin. Le dispositif selon l'invention comprend un simple cathéter 8 présentant un alésage longitudinal. L'extrémité distale 6 du cathéter 8 porte un corps souple et gonflable 10, fixé par tout moyen adéquat sur ladite extrémité et qui peut avoir la forme d'un ballon du genre que l'on emploie avec des cathéters utilisés pour exécuter des traitements dans les vaisseaux sanguins. Le ballon 10 peut être d'un type ayant un degré de flexibilité élevé, et ne doit pas nécessairement produire une dilatation importante du vaisseau 2.

Dans le cathéter 8, est inséré un élément de transmission du rayonnement laser, composé d'une fibre optique 12 entourée d'une couche de revêtement 14. La couche de revêtement 14 peut être de tout type appropriée, choisie pour confiner le rayonnement laser à l'intérieur de la fibre 12. L'extrémité distale de la fibre 12 se prolonge dans la région ou zone enveloppée par le corps gonflable 10 et peut avoir la forme d'une lentille convergente 16. L'extrémité proximale de la fibre 12 est raccordée à une source de lumière laser 20 capable de produire des impulsions de rayon laser d'une durée et d'un niveau d'énergie convenables.

Comme exposé dans le document US-A-5.116.227, la fibre optique 12 peut avoir un diamètre de l'ordre de 150 µ, et les impulsions du rayonnement produites par la source laser 20 peuvent avoir une énergie par impulsion de l'ordre de 5-200mJ, des niveaux d'énergie plus élevés étant utilisés de préférence pour des vaisseaux de diamètre plus large, une fréquence d'impulsions de 30-100 Hz et une durée d'impulsion de 10 ns à quelques ms.

A l'extrémité proximale du cathéter 8, l'alésage longitudinal de ce dernier est accouplé à une source d'alimentation en liquide 22, permettant d'envoyer un volume d'un liquide adéquat, tel que de l'eau ou une solution saline, à l'intérieur du corps gonflable 10. Le liquide fourni par la source 22 est de préférence refroidi, afin de produire une absorption efficace de la chaleur.

Si on le souhaite, l'intérieur du cathéter 8 peut être divisé, par exemple par une plaque bissectrice (non représentée), en vue de contenir deux trajets d'écoulement, pour permettre la circulation du liquide de refroidissement. Toutefois, dans de nombreux cas, le liquide peut avoir, à l'origine, une température suffisamment basse et une capacité d'absorption de la chaleur suffisante, pour permettre d'éviter la nécessité d'une telle circulation.

Le dispositif représenté sur la figure 1 peut être utilisé de la manière suivante : le cathéter 8 est inséré dans le vaisseau sanguin ou autre passage alors que le corps gonflable 10 se trouve à l'état dégonflé, c'est-à-dire avec l'intérieur du corps 10 et l'alésage du cathéter 8 mis sous vide, de sorte qu'il règne une dépression à l'intérieur de l'alésage, l'insertion dudit cathérer s'effectuant de manière à amener ledit corps 10 à l'état dégonflé en face du dépôt 4 à éliminer. Ensuite, l'alésage longitudinal du cathéter 8 et l'intérieur du corps 10 sont remplis de liquide de refroidissement en provenance de la source d'approvisionnement 22, le liquide étant fourni en quantité suffisante pour gonfler le corps 10 jusqu'à ce qu'il prenne la forme montrée sur la figure 1, dans laquelle la paroi du corps 10 vient en appui contre la surface exposée du dépôt 4, sans produire de force substantielle qui provoquerait une dilatation radiale du vaisseau 2. La fibre optique 12 est ensuite insérée à travers l'alésage longitudinal du cathéter 8, jusqu'à ce que son extrémité distale soit disposée dans la région ou zone délimitée par le corps 10. Ensuite, des impulsions de rayon laser sont produites par le laser 20, et dirigées vers l'extrémité distale de la fibre 12, et le rayonnement est focalisé en un point dans le liquide qui remplit le corps 10. L'énergie de chaque pulsation de rayon laser ainsi focalisée provoque une formation et une cavitation de vapeur au sein du liquide, ce qui entraîne l'implosion de bulles de gaz à la fin de chaque impulsion de rayon laser. L'agitation vigoureuse du liquide de refroidissement qui en résulte est transmise à la paroi du corps 10, et donc aux régions du dépôt 4 en contact avec la paroi du corps 10, ce qui entraine la désintégration du dépôt.

A mesure que la masse du dépôt 4 se réduit, la pression du liquide de refroidissement contenu dans le corps 10 amène la paroi de ce corps à rester en contact avec le reste de la matière du dépôt, ce qui fait que le processus de désintégration se poursuit.

A la fin du traitement, le liquide de refroidissement peut être évacué de l'intérieur du corps 10, pour faciliter le retrait du cathéter 8 dans le vaisseau 2.

On appréciera que la fibre 12 peut être placée dans le cathéter 8 avant l'insertion de ce dernier dans le vaisseau 2.

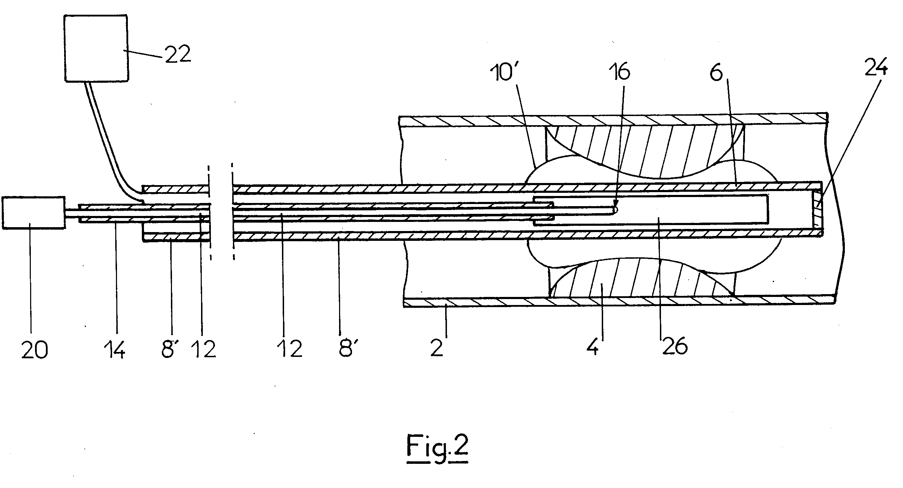

La figure 2 illustre un autre mode de réalisation de l'invention, qui comprend un cathéter 8' qui diffère du cathéter 8 de la figure 1 en ce que l'alésage longitudinal dudit cathéter 8' est fermé, à l'extrémité distale de ce dernier, par une paroi 24, et en ce que la paroi latérale dudit cathéter 8' est percée d'une pluralité d'ouvertures 26.

Le corps 10 de la figure 1 est remplacé, selon ce mode d'exécution, par un corps souple et gonflable 10', en forme de manchette annulaire, dont l'intérieur communique par lesdites ouvertures 26 avec l'alésage longitudinal du cathéter 8', pour fournir du liquide de refroidissement à la région enveloppée par ledit corps 10'.

La mise en oeuvre du dispositif représenté sur la figure 2 est identique à celle décrité précédemment en relation avec la figure 1.

Le dispositif selon l'invention offre l'avantage de réduire au maximum les dommages thermiques susceptibles d'être causés au vaisseau 2, et permet de transmettre avec précision au dépôt 4 à supprimer, les forces mécaniques produites par les rayons pulsés, focalisés dans le liquide de refroidissement. Le gonflage du corps 10 ou 10' peut être contrôlé, en vue de minimiser le transfert de ces forces mécaniques au vaisseau 2.

Bien que l'on puisse utiliser, pour la mise en oeuvre du dispositif et du procédé selon l'invention, des dispositifs à laser 20 qui produisent un rayonnement laser à différentes longueurs d'ondes, l'utilisation d'un laser YAG:Nd s'avère plus particulièrement appropriée. Un tel laser peut être régulé de manière à produire un rayonnement à une longueur d'onde de 0,53, 0,75 ou 1,06µ. On pourrait également avoir recours à un laser à rubis, dont le rayonnement à une longueur d'onde de 0,694µ.

Le liquide de refroidissement peut être d'une composition substantiellement transparente à la longueur d'onde du rayon laser. Toutefois, la focalisation du rayon laser, essentiellement en un point dans le liquide, engendre une intensité lumineuse suffisamment élevée pour produire le phénomène de cavitation et d'implosion décrit précédemment.

Bien que la description qui précède fait référence à des modes de réalisation particuliers de la présente invention, on comprendra que de nombreuses modifications peuvent être faites sans sortir de l'esprit de l'invention. Les revendications annexées sont réputées couvrir toute modification de ce genre, qui tomberait dans le cadre exact et l'esprit de la présente invention.

Les modes de réalisation présentement décrits doivent donc être considérés à tous égards comme illustratifs, et non limitatifs, le cadre de l'invention étant défini par les revendications annexées, plutôt que par la description qui précède, et tous les changements qui tombent sous le sens et dans le domaine d'équivalence des revendications sont donc réputés compris dans ces dernières.

## Revendications

1. Dispositif pour désintégrer un dépôt sur une surface intérieure d'un passage tubulaire susceptible d'être endommagé lorsqu'il est exposé à la chaleur,
caractérisé en ce qu'il comprend :
- un tube creux (8, 8') qui présente une ouverture et qui peut être inséré dans le passage (2), de sorte que l'ouverture soit placée à proximité du dépôt ;
- un corps souple et gonflable (10, 10'), constitué d'une feuille de matière fixée audit tube, de manière à fermée ladite ouverture ;
- une fibre optique (12) placée dans ledit tube (8,8') et présentant une extrémité distale à proximité dudit corps gonflable (10, 10') ;
- des moyens (22) pour introduire un volume de liquide dans ledit tube (8, 8') et l'amener en contact avec ledit corps gonflable (10, 10') , à une pression suffisante pour gonfler ce dernier de manière à le mettre en contact avec ledit dépôt (4), et
- des moyens (20) pour envoyer une succession d'impulsions d'un rayon laser à ladite fibre optique (12).

2. Dispositif suivant la revendication 1 caractérisé en ce que ledit corps souple et gonflable (10, 10') est constitué par un ballon.

3. Dispositif suivant l'une des revendications 1 ou 2, caractérisé en ce que ledit tube creux présente une extrémité distale (6), ladite ouverture ménagée dans ledit tube creux étant située au niveau de ladite extrémité distale (6) et en ce que le corps souple et gonflable (10) est fixé sur cette dernière.

4. Dispositif suivant la revendication 1 caractérisé en ce qu'il comprend une masse de liquide, raccordéeaux moyens d'introduction précités.

5. Dispositif suivant la revendication 4, caractérisé en ce que ladite masse de liquide est de l'eau ou une solution saline.

6. Dispositif suivant la revendication 1 caractérisé en ce que le tube creux (8') présente une paroi latérale et une extrémité distale fermée (24) ladite paroi latérale étant pourvue d'au moins une ouverture (26), à proximité de ladite extrémité distale, et en ce que le corps souple et gonflable précité (10') est constitué d'une manchette annulaire fixée à la paroi latérale dudit tube, de manière à être en communication avec ladite ouverture et à l'envelopper complètement.

7. Dispositif suivant l'une des revendications 1 ou 2, caractérisé en ce que ladite fibre optique (12) est pourvue, à son extrémité distale, d'une lentille convergente (16) destinée à focaliser le rayon laser dans la région enveloppée par ledit corps (10, 10').

8. Procédé pour désintégrer un dépôt formé sur une surface intérieure d'un passage tubulaire susceptible d'être endommagé lorsqu'il est exposé à la chaleur, avec utilisation du dispositif défini dans la revendication 1, caractérisé en ce qu'il consiste :
- à insérer le tube creux (8) dans le passage (2) de sorte que le corps souple (10) soit placé à proximité du dépôt (4) ;
- à remplir le corps gonflable (10) avec un volume de liquide, pour gonfler ledit corps (10) de manière à l'amener en contact avec le dépôt (4) ;
- à insérer la fibre optique (12) dans le tube (8) de manière à ce que l'extrémité distale de ladite fibre optique se situe à l'intérieur du corps gonflable (10); et
- à envoyer une succession d'impulsions de rayon laser à la fibre optique (12), de manière à faire sortir le rayon laser à l'extrémité distale de la fibre, et le faire pénétrer dans le liquide remplissant le corps (10).

9. Procédé suivant la revendication 8, caractérisé en ce que le rayon laser est focalisé en un point situé à l'intérieur du liquide remplissant le corps (10) et au-delà de l'extrémité distale de la fibre optique (12).

10. Procédé suivant l'une des revendications 8 ou 9 caractérisé en ce que le passage tubulaire (2) est un vaisseau sanguin.
